Europäisches Patentamt

European Patent Office

Office européen des brevets

(11) Publication number: **0 211 321**
**A2**

## EUROPEAN PATENT APPLICATION

(21) Application number: **86110081.6**

(22) Date of filing: **22.07.86**

(51) Int. Cl.4: **C12P 21/02** , **C12N 15/00** ,
**A61K 37/02**

The applicant has filed a statement in accordance with Rule 28 (4) EPC (issue of a sample only to an expert).

The title of the invention has been amended (Guidelines for Examination in the EPO, A-III, 7.3).

(30) Priority: **23.07.85 JP 162601/85**

(43) Date of publication of application:
**25.02.87 Bulletin 87/09**

(84) Designated Contracting States:
**AT BE CH DE FR GB IT LI LU NL SE**

(71) Applicant: **MOCHIDA PHARMACEUTICAL CO., LTD.**
**7, Yotsuya 1-chome**
**Shinjuku-ku Tokyo 160(JP)**
Applicant: **Hayshibara Biochemical Laboratories Incorporated**
**2-3, Shimoishii 1-chome**
**Okayama-shi Okayama-ken(JP)**

(72) Inventor: **Nobuhara, Masahiro**
**572-10, Yajuro**
**Koshigaya-shi Saitama-ken(JP)**
Inventor: **Nagase, Yasukazu**
**2-504, 1-16, Akabane Minami, Kita-ku Tokyo(JP)**

(74) Representative: **Hoffmann, Klaus, Dr. rer. nat. et al**
**Hoffmann . Eitle & Partner Patentanwälte**
**Arabellastrasse 4**
**D-8000 München 81(DE)**

(54) **Amino-acid composition, method for its production and medical composition containing it.**

(57) An amino acid composition comprising a specified amino acid sequence, showing a molecualr weight of 16,000 to 20,000 daltons upon SDS-polyacrylamide gel electrophoresis and 30,000 to 45,000 daltons upon gel filtration, and showing a specific activity of $0.5 \times 10^9$ to $3 \times 10^9$ units/mg protein. The amino acid composition exhibits an antitumor activity without injuring normal cells. A process of producing the amino acid composition via gene recombination technique and medical compositions thereof are also disclosed.

# AMINO ACID COMPOSITION, METHOD FOR PRODUCTION THEREOF AND MEDICAL COMPOSITION CONTAINING SAID AMINO ACID COMPOSITION AND THE USE THEREOF

## BACKGROUND OF THE INVENTION

The present invention relates to a novel amino acid composition and a method for producing the same via genetic recombination technique, as well as a medical composition comprising the amino acid composition as the effective component and the use thereof.

It is hitherto known that lymphocytes produce lymphokines having cytotoxic activity and there are a large number of reports on substances having cytotoxic activity. However, very few substances of high purity have actually been determined on their physical and chemical properties. Most of those which have been determined were determined on physical and chemical properties or biological activities in such a state that many kinds of contaminants were present in large quantities.

## SUMMARY OF THE INVENTION

As a result of extensive investigations with an attempt to produce an anti-tumor substance produced by human tissue cells as a single substance in view of the foregoing actual situation, the present inventors have come to provide a novel amino acid composition which was hitherto unknown.

Another object of the present invention is to provide a method for producing the aforesaid novel amino acid composition using human lymphatic cells via genetic recombination techniques.

A further object of the present invention is to provide a medical composition comprising as an effective ingredient the aforesaid novel amino acid composition.

## BRIEF DESCRIPTION OF THE DRAWINGS

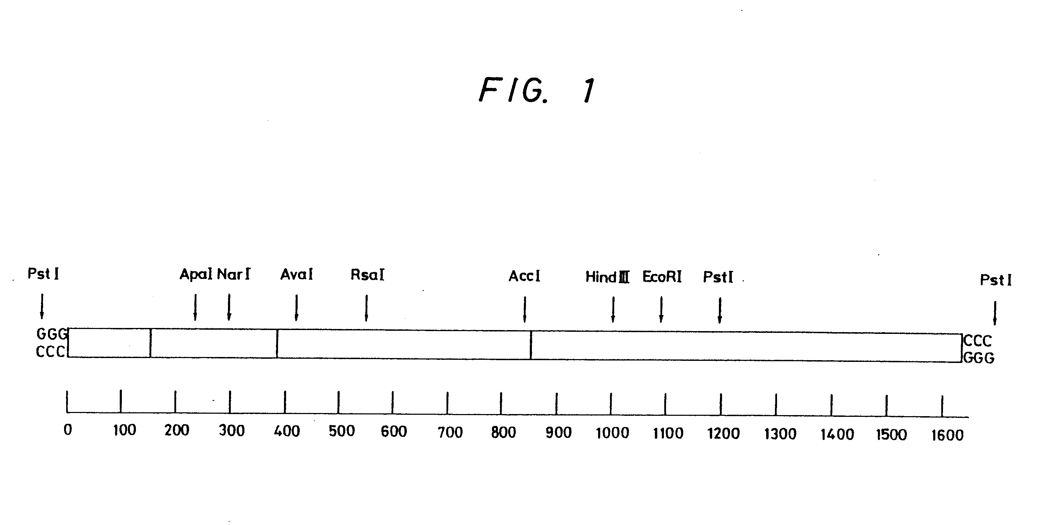

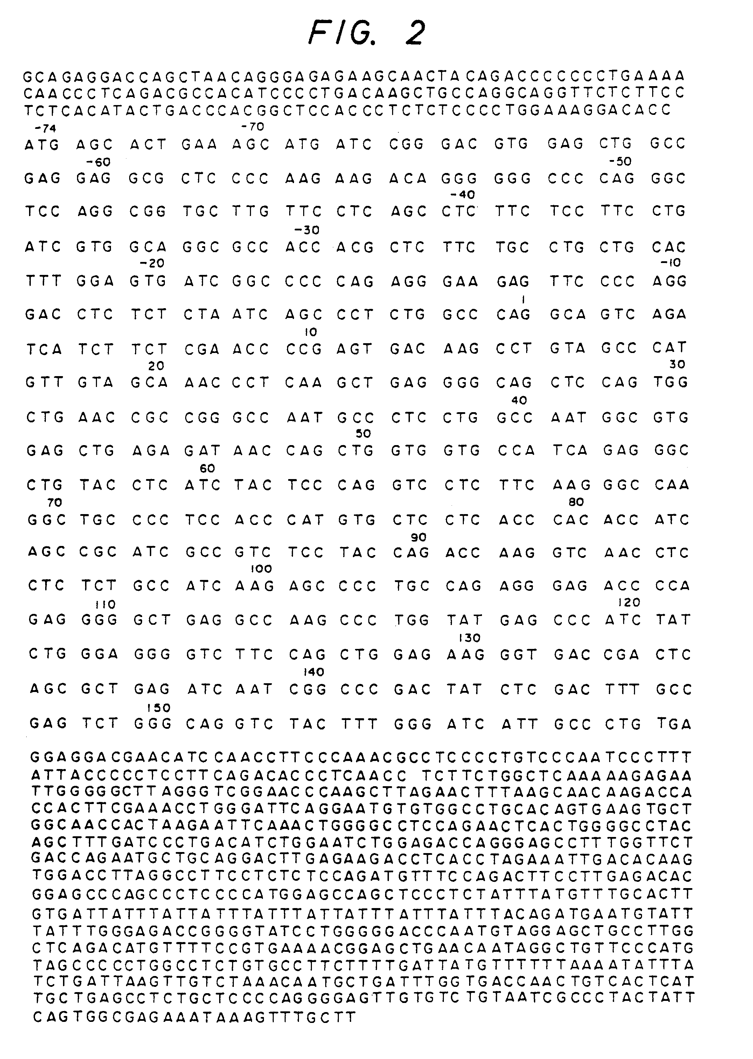

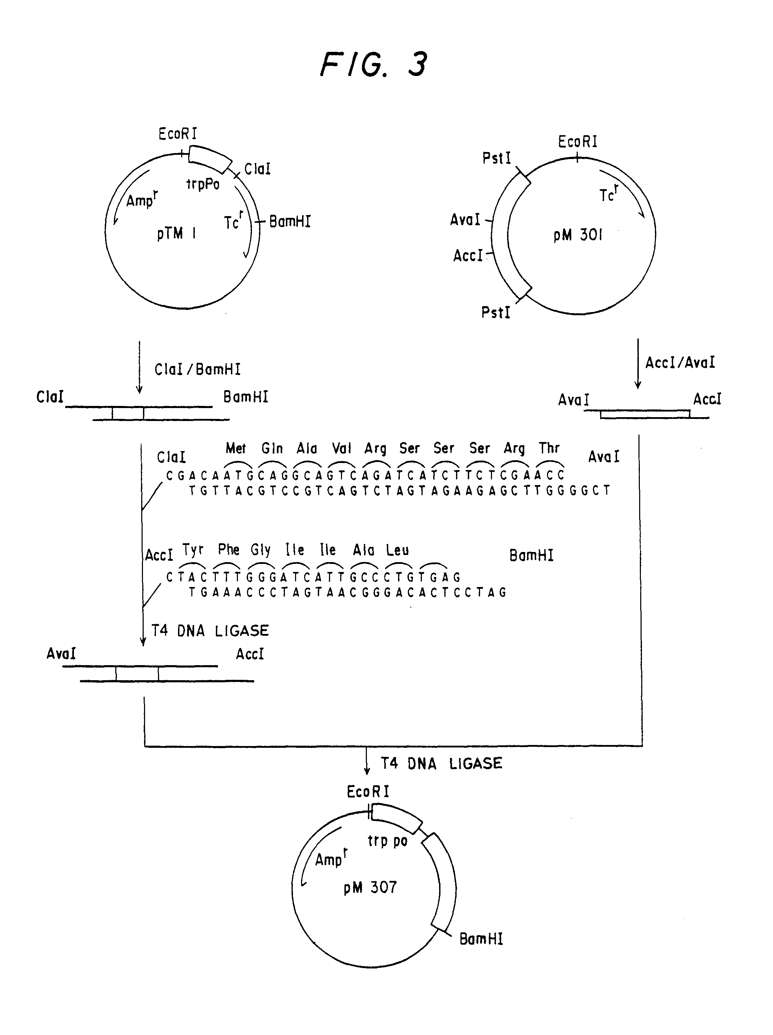

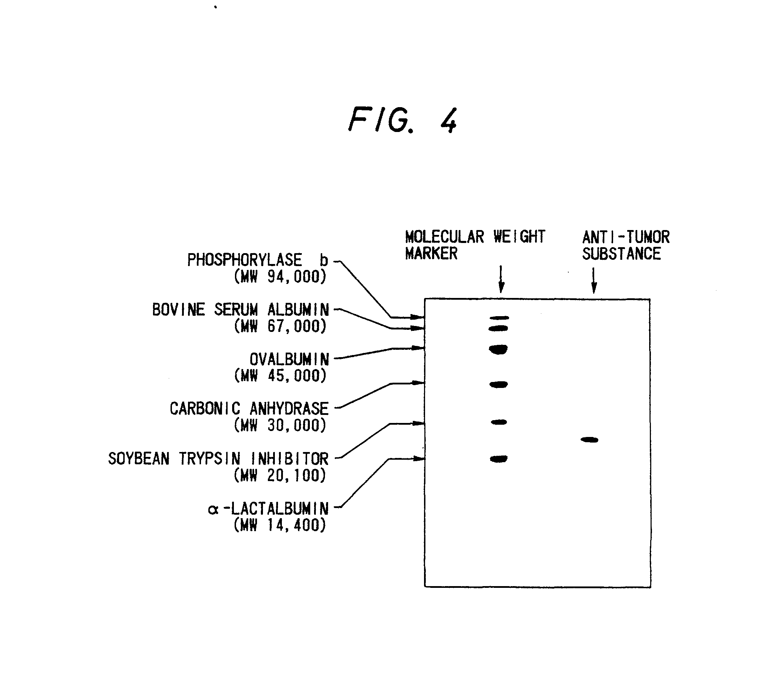

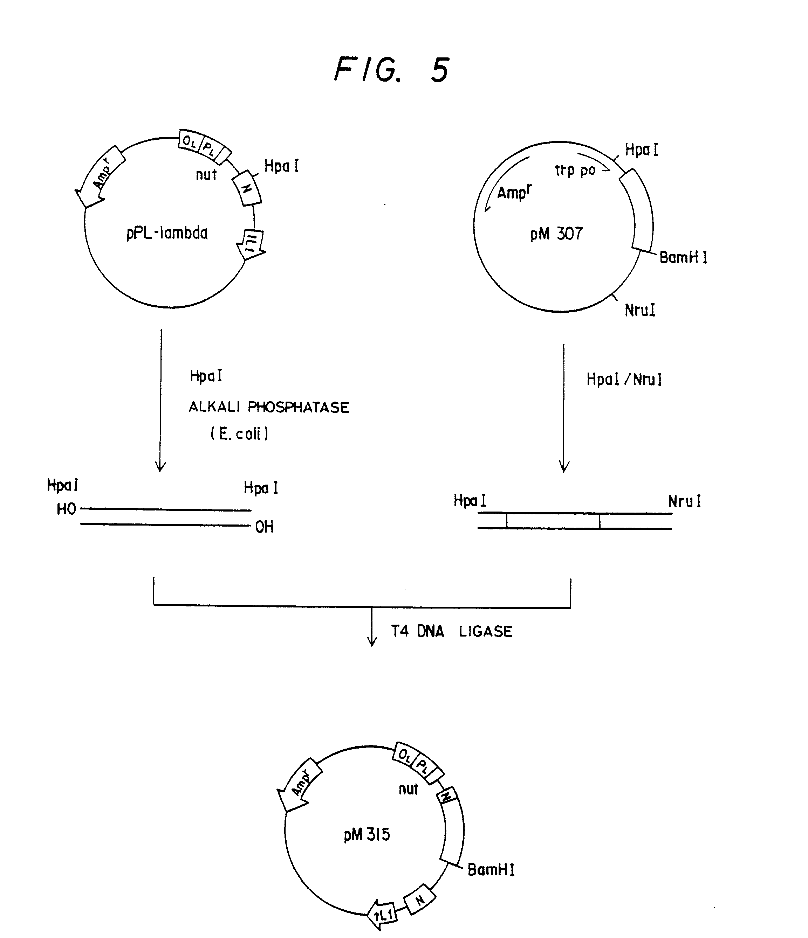

Figure 1 shows the restriction map of plasmid pM 301 obtained in Example 1 (V). Figure 2 shows that primary structure (DNA sequence) of plasmid pM 301 obtained in Example 1 (V). Figures 3 and 4 respectively show the construction map and results of electrophoresis of the novel amino acid composition of the present invention obtained in Example 2. Figure 5, Figures 6 and 7 and Figure 8 show the construction maps of pM 315 of Example 3, pM 389 of Example 4 and pM 390 of Example 5, respectively.

## DETAILED DESCRIPTION OF PREFERRED EMBODIMENTS

The present invention attains the foregoing and other objects and relates to the novel amino acid composition, and its production and use.

The amino acid composition of the present invention is characterized by its amino acid sequence and characteristic properties. Namely, the amino acid sequence is as described below:

H-Gln Ala Val Arg Ser Ser Ser Arg Thr Pro Ser Asp Lys Pro Val Ala His Val Val Ala Asn Pro Gln Ala Glu Gly Gln Leu Gln Trp Leu Asn Arg Arg Ala Asn Ala Leu Leu Ala Asn Gly Val Glu Leu Arg Asp Asn Gln Leu Val Val Pro Ser Glu Gly Leu Tyr Leu Ile Tyr Ser Gln Val Leu Phe Lys Gly Gln Gly Cys Pro Ser Thr His Val Leu Leu Thr His Thr Ile Ser Arg Ile Ala Val Ser Tyr Gln Thr Lys Val Asn Leu Leu Ser Ala Ile Lys Ser Pro Cys Gln Arg Glu Thr Pro Glu Gly Ala Glu Ala Lys Pro Trp Tyr Glu Pro Ile Tyr Leu Gly Gly Val Phe Gln Leu Glu Lys Gly Asp Arg Leu Ser Ala Glu Ile Asn Arg Pro Asp Tyr Leu Asp Phe Ala Glu Ser Gly Gln Val Tyr Phe Gly Ile Ile Ala Leu-OH

The typical properties of the amino acid composition are as follows:

(a) The molecuar weight is in the range of 16,000 to 20,000 daltons when determined according to SDS-polyacryl-amide gel electrophoresis and in the range of 30,000 to 45,000 daltons when determined by gel filtration.

(b) It has a cytotoxic activity against L929 cells in vitro and has a specific activity of $0.5 \times 10^9$ to $3 \times 10^9$ units/mg protein.

The amino acid composition of the present invention comprising the above described amino acid sequence is a substantially single novel substance and is particularly useful for medicament as later described.

Further, the method for producing the aforesaid amino acid composition of the present invention is characterized by isolating the cytoplasmic RNA from a human lymphatic cell, isolating the mRNA from the cytoplasmic RNA, producing a single-stranded complementary DNA (cDNA) using the mRNA as a template, then producing a double-stranded cDNA with the single-stranded cDNA, inserting the double-stranded cDNA into a plasmid vector, transforming a bacterial cell with the cDNA vector wherein the double-stranded cDNA has been inserted isolating the plasmid DNA from the cDNA clone that is positive to the probe, determining the DNA sequence of the plasmid DNA, inserting the DNA into an appropriate expression vector, transforming an appropriate host cell with the expression vector and then culturing the host cell.

When it is desirable that the obtained amino acid composition be a substantially single substance, the production method may contain the steps of isolating the amino acid composition from the culture of the host cell and purifying the composition. The aforesaid amino acid composition of the present invention is useful as a medical composition for treating human tumors since said amino acid composition has the above-described properties and shows remarkable anti-tumor activity against various kinds of tumor cells as later described.

The following will explain the present invention and its effects in detail. The abbreviations used in this specification are based on conventional abbreviations in the art, some examples of which are shown below. In case that optical isomers can be present with respect to amino acid, etc., they are in the L-form unless otherwise indicated.

DNA :deoxyribonucleic acid

RNA :ribonucleic acid

A :adenine

T :thymine

G :guanine

C :cytosine

dATP :deoxyadenosine triphosphate

dTTP :deoxythymidine triphosphate

dGTP :deoxyguanosine triphosphate

dCTP :deoxycytidine triphosphate

ATP :adenosine triphosphate

EDTA :ethylenediaminetetraacetate

SDS :sodium dodecylsulfate

Ala :alanine

Arg :arginine

Asn :asparagine

Asp :aspartic acid

Cys :cystein

Gln :glutamine

Glu :glutamic acid

Gly :glycine

His :histidine

Ile :isoleucine

Leu :leucine

Lys :lysine

Met :methionine

Phe :phenylalanine

Pro :proline

Ser :serine

Thr :threonine

Trp :tryptophane

Tyr :tyrosine

Val :Valine

The numerals with parentheses for descriptions on known methods, etc., as used in the specification refer to publications corresponding to the numerals described at the end of the specification.

The DNA coding for the aforesaid amino acid composition of the present invention contains, e.g., the following base sequence:

CAG GCA GTC AGA TCA TCT TCT CGA ACC
CCG AGT GAC AAG CCT GTA GCC CAT GTT
GTA GCA AAC CCT CAA GCT GAG GGG CAG
CTC CAG TGG CTG AAC CGC CGG GCC AAT
GCC CTC CTG GCC AAT GGC GTG GAG CTG
AGA GAT AAC CAG CTG GTG GTG CCA TCA
GAG GGC CTG TAC CTC ATC TAC TCC CAG
GTC CTC TTC AAG GGC CAA GGC TGC CCC
TCC ACC CAT GTG CTC CTC ACC CAC ACC
ATC AGC CGC ATC GCC GTC TCC TAC CAG
ACC AAG GTC AAC CTC CTC TCT GCC ATC
AAG AGC CCC TGC CAG AGG GAG ACC CCA
GAG GGG GCT GAG GCC AAG CCC TGG TAT
GAG CCC ATC TAT CAG GGA GGG GTC TTC
CAG CTG GAG AAG GGT GAC CGA CTC AGC
GCT GAG ATC AAT CGG CCC GAC TAT CTC
GAC TTT GCC GAG TCT GGG CAG GTC TAC
TTT GGG ATC ATT GCC CTG TGA

And the foregoing DNA may also contain ATG or the following base sequence, at the 5'-terminal end thereof:

ATG AGC ACT GAA AGC ATG ATC CGG GAC
GTG GAG CTC GCC GAG GAG GCG CTC CCC
AAG AAG ACA GGG GGG CCC CAG GGC TCC
AGG CGG TGC TTG TTC CTC AGC CTC TTC
TCC TTC CTG ATC GTG GCA GGC GCC ACC
ACG CTC TTC TGC CTG CTG CAC TTT GGA
GTG ATC GGC CCC CAG AGG GAA GAG TTC
CCC AGG GAC CTC TCT CTA ATC AGC CCT
CTG GCC

Isolation of mRNA

To produce the aforesaid mRNA according to the method of the present invention, any cells can be used as far as they are those which are capable of producing the desired amino acid composition. Among them, BALL-1 cells (1) are preferred because of its high productivity of the desired substance.

BALL-1 cells can be proliferated not only in vitro but also in a non-human warm-blooded animal, e.g., hamster, mouse, rat or guinea pig, by transplanting the cell in the animal after immunosuppression. These procedures are applicable to other cell lines. Regardless of the proliferation methods, the obtained cells can be used to isolate the mRNAs coding the amino acid composition according to the present invention. The aforesaid cell lines continue to produce the amino acid composition but its amount may be small. The amino acid composition productivity of lymphatic cells can be enhanced with (A) specific antigens, e.g., phytohemagglutinin, concanavalin A and porkweed mitogen, that act as the mitogen on lymphocytes, (B) carcinogenetic promoters such as phorbol ester and others, (C) nucleic acids such as poly I : poly c, (D) substances that give a biological stimulus to lymphocytes such as viruses. Among these substances, viruses are preferable for isolation of the mRNAs because viruses enhance the production of the amino acid composition. Any virus can be used as far as it enhances the production of the amino acid composition. Specifically suitable viruses are RNA viruses such as Sendai virus and Newcastle disease virus. Among them, Sendai virus is most preferable.

Isolation of the mRNAs that code the aforesaid amino acid compsition from BALL-1 cell can be generally carried out as follows: the cells obtained by proliferating BALL-1 cells in a non-human warm-blooded animal such as immunosuppressed hamsters, or by culturing BALL-1 cells in a medium containing 20% (V/V) or much less serum, e.g.,

RPMI 1640 medium, for 1 to 6 days can be used to isolate the mRNAs. After washing with a serum-free culture medium one to 5 times, the cells are suspended in a serum-free culture medium, e.g., RPMI 1640 medium, to give a cell density of $1 \times 10^5$ to $2 \times 10^5$/ml, followed by cultivation. As the culture vessel, conventional culture devices such as Roux flasks, roller bottles, and spinner incubators can be used. The most preferable is the spinner incubator. Cultivation can be carried out at temperature in the range of 30 to 39°C, preferably 37°C. When, for example, Sendai virus is used, the cultivation is continued for 1 to 7 days, but this varies dependent upon the presence and concentration of the virus. Sendai virus is generally incorporated to a concentration ranging from 1 to 10,000 HAU/ml. The concentration range from 10 to 1,000 HAU/ml is most preferable because such range enhance the production of the amino acid composition.

After completion of the culture, the cells are collected by centrifugation. A ribonuclease inhibitor selected from guanidine thiocyanate, vanadium complex, heparin, diethyl pyrocarbonate and sodium dodecylsulfate is incorporated, and a member selected from N-laurylsarcosine, Tween 80 and Nonidet P-40 is added to the cells to extract RNAs. If necessary, phenol or a mixture thereof with chloroform may be added to the extract containing the RNAs, and this extraction may be repeatedly carried out. Then, the RNAs are collected by precipitation with ethanol.

Thereafter the poly A+ RNAs are collected with oligo dT cellulose, poly U Sepharose, or the like. Since most of the eucaryocytic DNAs bear the poly A at the 3'-terminal end thereof, the mRNAs can be selectively harvested from the whole RNAs by this operation. By repeating this operation, the purity of the mRNA can be further enhanced, if necessary.

Preparation of cDNA Library

Using the thus obtained mRNA as the template, single-stranded cDNAs are synthesized by conventional methods using reverse transcriptase and then converted into double-stranded form. To synthesize the double-stranded cDNAs, conventional methods using S1 nuclease (2) and that reported by Land et al. (3) can be employed. A sticky end is formed at the 3'-terminal end of the double-stranded cDNAs with terminal transferase, and incorporated into a restriction site of the plasmid having a drug resistance. for this purpose, the Pst I site of plasmid pBR322 can be advantageously used. It is also possible to incorporate the double-stranded cDNAs into the plasmid with the method reported by Okayama et al. (4).

Using the obtained plasmid, bacterial cells, specifically those of Escherichia coli, are transformed by conventional methods to prepare a cDNA library. For this purpose, the use of Escherichia coli χ 1776 strain (American Type Culture Collection (ATCC) 31244) is preferable.

Screening of cDNA

After digesting the culture of a cell capable of producing the amino acid composition of the present invention with proteases such as trypsin or lysyl endopeptidase, the resultant fragments are fractionated by high performance liquid chromatography (HPLC) and their amino acid sequences are determined according to the method of Hewick et al. (5) where a gaseous phase protein sequencer - (Model 470, Applied Biosystems Inc.) is used.

Based on the obtained amino acid sequence, a mixture of 8 to 64 different oligonucleotides having a DNA sequence coding for the amino acid sequence is synthesized with a full automatic DNA synthesizer (Model 380 A, Applied Biosystems Inc.).

The 5'-terminal end of the oligonucleotide mixture is then labelled with $^{32}$P with an appropriate means to prepare a probe.

Using this probe, the previously obtained cDNA library is screened by a conventional colony hybridization method (6). The DNAs of clones that were positive against the known colony hybridization method are isolated and the DNA sequence coding for the amino acid composition of the present invention is determined by the dideoxy chain termination method using phage M13 (7) or the Maxam-Gilbert method (8).

Expression

The gene with the DNA sequence as determined above is fragmented with an appropriate restriction enzyme. Separately, in order to ligate the gene with a promoter of expression plasmid at the downstream thereof, the linkers are synthesized with a full automatic DNA synthesizer (Model 308 A, Applied Biosystems Inc.). Both DNA fragments are ligated together with an enzyme such as T4 DNA ligase, to insert the DNA fragments into the promoter of expression plasmid at the downstream thereof. In this way, an appropriate host can be transformed. After proliferating the transformed cell in a suitable culture medium, the amino acid composition of the present invention can be produced, if necessary, via an appropriate induction step.

Hosts for producing the amino acid composition of the present invention include Escherichia coli, Bacillus subtilis, yeast, and mammalian cells including those of human origin. When Escherichia coli HB 101 strain (ATCC 33694), W 3110 strain - (ATCC 27325), C 600 strain (ATCC 23724), MM 294 strain (ATCC 33625), or DH 1 strain (ATCC 33849) is used as the host, tryptophane (Trp) promoter, lactose (Lac) promoter, elongation factor Tu (Tuf B) promoter or PL promoter of λ phage can be used as the promoter. Among them, the most preferable is the Trp promoter. When a yeast such as Saccharomyces cerevisiae is used as the host, glycolytic enzyme promoters including acid phosphatase promoter, 3-phosphoglycerate kinase promoter and alcohol dehydrogenase promoter can be used as the promoter. The mammalian cell such as Chinese hamster ovary (CHO) cells, monkey COS cells (ATCC CRL 1650, CRL 1651), mouse C 127 cells (ATCC CRL 1616) can be used as the host cells. The promoters that can be used in such mammalian cells are the early and later promoters of sarcoma virus 40 (SV 40), and the metallothioneine promoter.

Purification

In order efficiently to obtain the amino acid composition of the present invention in a high purity, a mouse monoclonal antibody that specifically binds the amino acid composition of the present invention and neutralizes the cytotoxic activity of the composition was prepared (9). Particularly, a solution of a crude amino acid composition obtained by culturing BALL-1 cells was subjected to ultrafiltration using PM 10 Membrane (Amicon Co., Ltd.) and then partially purified by gel filtration chromatography using Sephadex G-75. The fractions with a molecular weight of 30,000 to 45,000 daltons and containing the amino acid composition were collected from the eluates. After concentrating the fractions using PM 10 Membrane, the concentrate was used together with Freund's complete adjuvant to immunize a BALB/c mouse. After an additional immunization, the mouse spleen was isolated from the body of the mouse to prepare a suspension of the spleen cells. The spleen cells were then fused with SP 2/0-Ag 14 cells (Dainippon Pharmaceutical Co., Ltd.) using polyethylene glycol 1540 (Wako Junyaku Kogyo Co., Ltd.) in a conventional manner. After fusion, the obtained cell colonies were selected with HAT (Hypoxanthine, Aminopterin, Thymidine) to obtain a hybridoma capable of producing an antibody that neutralizes the cytotoxic activity of the amino acid composition of the present invention. The monoclonal antibody produced by this hybridoma is of class IgM and can be purified in a conventional manner. The cytotoxic activity was detected only in the liquid obtained by eluting the column with 50mM ethylamine hydrochloride buffer (pH 11.0) when the amino acid composition solution used as the immunogen was passed through a column of Sepharose 4B resin binding the monoclonal antibody. This confirmed that the antibody was a monoclonal antibody specific to the amino acid composition of the present invention.

It is desirable to purify this monoclonal antibody by salting out with ammonium sulfate or ion exhange chromatography prior to its use. The monoclonal antibody can be used as a ligand by binding it on such as cyanogenebromide-activated Sepharose 4B (Pharmacia Japan Co.) or Affigel-10 (Japan Biorad Laboratories) with a spacer. Although the adsorbing of the amino acid composition can be effected batchwise, it is more efficient to use a column of the carrier. The adsorption can be performed in a neutral region, preferably under buffering conditions using a phosphate buffer of pH 6.0 to 8.0. Although the amino acid composition can be eluted from the carrier with an acidic eluting solution such as 0.1 M glycine-hydrochloride (pH 2.0), it is preferred to use a neutral buffer solution containing 8M urea solution, 60% ethylene glycol solution, 60% propylene glycol solution or 50mM ethylamine hydrochloride buffer (pH 11.0), due to the fact that the amino acid composition of the present invention is unstable in a low pH region. After thoroughly dialyzing the eluate against a neutral buffer solution, e.g., 0.14M sodium chloride-containing 0.01M phosphate buffer (pH 7.2), a portion of the dialyzed product was measured for cytotoxic activity against L929 cells to evaluate an activity recovery rate to that of the crude solution. The recovery rate is 50% or higher, generally higher than 80%. The specific activity of the product can be calculated by measuring the protein concentration of an aliquot of the dialyzed product. The specific activity is $0.1 \times 10^9$ to $5 \times 10^9$ units/mg protein, generally $0.5 \times 10^9$ to $3 \times 10^9$ units/mg protein. Upon staining with Coomassie Brilliant Blue the gel obtained by subjecting a part of the dialyzed product to SDS-polyachrylamide gel electrophoresis, a stained band is noted at a distance corresponding to a molecular weight of 16,000 to 20,000 daltons, but no additional clear band is generally noted. Scanning of the gel using a densitometer reveals that the purity of the amino acid composition is generally 95% or higher.

The amino acid composition of the present invention can be satisfactorily purified solely by using the monoclonal antibody-binding carrier, but this step can be combined with other purification procedures. Generally, in the purification using a monoclonal antibody-bound carrier, the antibody molecules may be liberated from the carrier. Contamination of a trace lipopolysaccharide having a potent pyrogenic action is well pointed out in purification steps in general. In order to remove the lipopolysaccharides, it is desirable to subsequently effect further purification procedures, for example, gel filtration chromatography.

In such gel filtration chromatography, one can use carriers such as Sephadex G-75, Sephadex G-100, Sepharose CL-48 (all available from Pharamacia Japan Co., Ltd.) and Biogel P-100 - (Japan Biorad Laboratories). As the developers for the gel filtration chromatography, any neutral solution can be used as long as it does not inactivate the amino acid composition and prevent its absorption onto the carrier. For example, 0.01M phosphate buffer (pH 7.2), containing 0.4M sodium chloride, can be used. When a gel filtration chromatography using, e.g., Sephadex G-75 is effectd after chromatography using a column of a monoclonal antibody-bound carrier to prepare a fraction with a molecular weight of 30,000 to 45,000 daltons, the recovery rate of the amino acid composition throughout the chromatography is 90% or higher, and the specific activity of the amino acid composition in the obtained fraction is generally $0.5 \times 10^9$ to $3 \times 10^9$ units/mg protein. SDS-polyacrylamide gel electrophoresis of the fraction after concentration gave only a single stained band of the amino acid composition at the location corresponding to a molecular weight of 16,000 to 20,000 daltons when not only stained with Coomassie Brilliant Blue but even with silver staining (10) that has a high detectability to proteins. The fact that only a single stained band appears by SDA-polyacrylamide gel electrophoresis performed after purification through two types of chromatographies showing different purification mechanisms reveals that the amino acid composition of the present invention has been purified to substantial homogeneity.

Although the step of using a carrier bearing the monoclonal antibody is capable of satisfactorily purifying the amino acid composition even though that step is solely performed, a much more satisfactory result can be obtained if other purification procedures are performed prior to the purification step using the carrier bearing the monoclonal antibody. As such purification procedure(s), conventional purification procedures can be adopted. For example, there can be used one or more of ultrafiltration, dialysis, salting out, lyophilization, centrifugation, extraction or precipitation with organic solvent, ion exchange chromatography, gel filtration chromatography, isoelectric point electrophoresis, isokinetic electrophoresis, chromatofocusing, polyacrylamide gel electrophoresis, and other chromatographies such as hydrophobic chromatography using phenyl Sepharose, and HPLC.

The present invention will be described in more detail with reference to the following examples, experiments, and application examples, but shall not to be limited thereto.

Since the aforesaid amino acid composition exhibits, typically, an anti-tumor activity it will be referred to as "anti-tumor substance" hereinafter.

## Example 1

### (I) Isolation of mRNA

Newborn hamsters were injected with anti-sera prepared from rabbit in conventional manner to weaken the immune reaction of the hamsters. Then BALL-1 cells were subcutaneously transplated followed by 3-week-feeding in conventional manner. The tumors formed subcutaneously in three hamsters were extracted, minced and disaggregated in serum-free RPMI 1640 medium. After washing once with serum-free RPMI 1640 medium, the cells were suspended in a fresh preparation of the same culture medium to $5 \times 10^6$/ml followed by 3-hour incubation at 37°C. Sendai virus was added to the culture medium to a final concentration of 100 HAU/ml followed by an additional 3-hour incubation at 34°C. After completion of the culture, the supernatant was removed by centrifugation to obtain $3 \times 10^9$ cells. The cells were suspended in a guanidine-modified solution (6M guanidine thiocyanate, 5mM sodium citrate, 0.5% sodium sarcosinate and 0.67% $\beta$-mercaptoethanol). The suspension was passed through an 18G injection needle to effect a destructive denaturation. The resultant homogeneous solution was put in a layer on 5.7M cesium chloride solution (5.7M cesium chloride, 0.1M EDTA), which was then subjected to centrifugation at 25°C for 18.5 hours at 35,000 rpm with a swing rotor, followed by collecton of the RNA precipitate. The RNA precipitate was dissolved in distilled water and precipitated with ethanol to obtain 7.1 mg of the RNA.

The RNA was adsorbed onto an oligo dT cellulose column in a solution of 0.5M NaCl, 10 mM Tris-HCl (pH 7.4), 1mM EDTA and 0.1% SDS. By treating the column with a solution of 10 mM Tris-HCl (pH 7.4), 1 mM EDTA and 0.1% SDS, mRNA containing 340g of poly A was obtained.

### (II) Synthesis of double-stranded cDNA

Nine $\mu$g of the mRNA, 3 Mg of oligo dT and 40 units of reverse transcriptase were incubated at 42°C for 90 minutes in 100 $\mu$l of a mixture solution (1 mM each of dATP, dCTP, dGTP and dTTP, 8 mM MgCl$_2$, 50 mM KCl, 0.4 mM dithiothreitol - (DTT) and 50 mM Tris-HCl (pH 8.3)) to synthesize single-stranded cDNAs. After removing the proteins with phenol, the RNA was decomposed by treatment with 0.1N NaOH at 37°C for 14 hours.

The single-stranded cDNAs were reacted at 37°C for five minutes in 20 $\mu$l of a mixture solution (0.14M potassium cacodylate, 30 mM Tris base, 0.1 mM DTT, 1 mM CoCl$_2$ and 0.05 mM dCTP) to extend them by about 15 deoxycytidine chains at the 3'-terminal end of the single-stranded cDNAs.

The cDNAs were reacted at 42° for 90 minutes in the same reaction system as used for synthesis of the single-stranded cDNAs using oligo dG and DNA polymerase I large fragments to synthesize double-stranded cDNAs.

The obtained double-stranded cDNAs were extended by about 10 deoxycytidine chains at the 3-terminal end by the same reaction system as in the deoxycytidine chain extension reaction of the single-stranded cDNA using terminal transferase.

### (III) Preparation of transformant

Ten $\mu$g of the obtained deoxycytidine chain-extended double-stranded cDNA and 25 $\mu$g of plasmid pBR 322 containing deoxyguanosine chains extended at the Pst I cleavage site - (Bethesda Research Laboratories, Inc.) were heated at 68°C for five minutes in 100 $\mu$l of a mixture solution (0.1 M NaCl, 10 mM Tris-HCl (pH 7.5) and 1 mM EDTA) and at 43°C for an additional 2 hours and then gradually cooled to 25°C. Escherichia coli $\chi$ 1776 strain was transformed in a conventonal manner to give about 80,000 transformants that were tetracycline-resistant.

### (IV) Determination of amino acid sequence

In order to determine the amino acid sequence of the anti-tumor substance, the following method was used.

100 $\mu$g of the anti-tumor substance produced by the BALL-1 cells was reacted with 1 $\mu$g of lysyl endopeptidase at 30°C for 24 hours in 50 mM of Tris-HCl (pH 9.2). Each of the resultant amino acid fragments was separated with HPLC using a reversed phase column. The amino acid sequence was determined with one of the separated amino acid fragments using a gaseous phase protein sequencer (Model 470 A, Applied Biosystems Inc.) in accordance with the method of Hewick et al. (5). The results were shown below using 3 character symbols representing amino acid residues by IUP-AC.

Pro-Trp-Tyr-Glu-Pro-Ile-Tyr-Leu-Gly-Gly-Val-Phe-Gln-Leu

The amino acid sequence of the anti-tumor substance at the amino terminal end was determined similarly as above with 50 μg of the anti-tumor substance. The results were as shown below.

Gln-Ala-Val-Arg-Ser-Ser-Arg-Thr-Pro-Ser-Asp-Lys-Pro-Val

(V) Determination of DNA sequence of cDNA

Based on the amino acid sequence obtained with the above-described method, the DNA sequence:

```
( 5' )  TGGTATGAACCTATTTA      ( 3' )
           C  G  C  C
                 A  A
                 G
```

deduced from the amino acid sequence of Trp-Tyr-Glu-Pro-Ile-Tyr was chemically synthesized with an automatic DNA synthesizer (Model 380 A, Applied Biosystems Inc.). The obtained oligonucleotide was exposed to the action of 6 units of T4 polynucleotide kinase at 37°C for 15 minutes in 25 μl of a mixture solution (50 mM Tris-HCl (pH 7.5), 10 mM MgCl$_2$, 1 mM DTT, 0.1 mM EDTA, 0.1 mM Spermidine and 200 μCi-$^{32}$P ATP) to label the 5'-terminal end with $^{32}$P.

Approximately 80,000 cDNA clones were fixed on a nitrocellulose filter and then screened with $^{32}$P-labeled oligonucleotide as the probe. Among five clones that were positive to the probe, the plasmid was isolated by the alkali method. Thereafter, the insertion parts of the plasmid DNA were excised with restriction enzyme Pst I to determine their length. The insertion part containing the longest fragment was designated as "pM 301", and its restriction cleavage map was prepared (Figure 1). Then, the DNA sequence of the cDNA inserted in the plasmid was determined by the M13 dideoxy chain termination method (Figure 2).

Example 2

Plasmid pTMl wherein the tryptophane synthesis promoter of Escherichia coli has been spliced into the Cla I cleavage site of plasmid pBR 322 was used as the expression vector. Plasmid pTMl was digested with restriction enzymes Cla I and Bamh I to give a DNA fragment of 4.3 kbp.

An oligonucleotide adaptor:

CGACAATGCAGGCAGTCAGATCATCTTCTCGAA-CC

TGTTACGTCCGTCAGTCTAGTAGAAGAGCTTGG-GGCT

containing a protein synthesis initiation codon ATG that had been chemically synthesized by the above-described method, and an oligonucleotide adaptor:

CTACTTTGGGATCATTGCCCTGTGAG

TGAAACCCTAGTAACGGGACACTCCTAG

containing a protein synthesis termination codon TGA, chemically synthesized by the aforesaid method, were ligated with the aforesaid DNA fragments of 4.3 kbp with T4 DNA ligase. Separately, the aforesaid pM 301 was digested with restriction enzymes Ava I and Acc I to recover a DNA fragment of about 430 bp containing most of the structural genes of the anti-tumor substance. The expression plasmid pM 307 for the anti-tumor substance was obtained with this DNA fragment and the aforesaid DNA containing the adaptors (Figure 3).

Using this plasmid, Escherichia coli HB 101 strain was transformed in a conventional manner to give transformant Escherichia coli HB 101/pM 307.

Transformant Escherichia coli HB 101/pM 307 was cultured at 37°C in mini jar fermenters (Model MB-C, Kabushiki Kaisha Iwashiya Seibutsu Kagaku) charged with 2 liters of M9 culture medium containing 50 μg/ml of ampicillin, 0.5% of casamino acid and 1% of glucose. When proliferation of the bacteria reached 1.0 in OD$_{550}$, 10 μg/ml of 3β-indolyacrylic acid (IAA) was added to the culture followed by an additional 4-hour incubation.

The obtained culture solution, 2 liters, was centrifuged and the bacterial cells were collected. The bacterial cells were suspended in 28 mM of EDTA containing 95 ml of 15% sucrose and 10 mM of Tris-HCl buffer (pH 8.0). After 5 ml of 10 mg/ml lisozyme solution in the same buffer (omitting sucrose) was added to the suspension, the mixture solution was then allowed to stand at 0°C for 30 minutes. Then, the mixture solution was treated at 0°C for 5 minutes with an ultrasonic homgenizer (Hitachi Seiki Co., Ltd.).

The cell lysate was centrifuged to obtain about 90 ml supernatant which was then purified with a carrier binding a monoclonal antibody to the anti-tumor substance in a manner as described below. A hybridoma capable of producing the monoclonal antibody specific to the anti-tumor substance was proliferated in RPMI 1640 medium, supplemented with 10% (v/v) bovine fetal serum, and then suspended in serum-free RPMI 1640 medium to 4 x 10⁶ cells/ml. After culturing at 37°C for 48 hours, the culture was harvested to obtain a crude solution of the monoclonal antibody. The crude solution was subjected to salting out with a 40% saturated ammonium sulfate solution and purified with a column of DEAE-Sephacel (Pharmacia Japan Co., Ltd.) to obtain a purified monoclonal antibody. After swelling bromocyanide-activated Sepharose 4B - (Pharmacia Japan Co., Ltd.) in a 1 mM hydrochloric acid solution, 80 mg of the purified monoclonal antibody were bound onto 50 ml of the Sepharose 4B using a 0.1M carbonate buffer (pH 8.3) containing 0.5M sodium chloride as the coupling buffer. After the unreacted groups were blocked with glycine, the Sepharose 4B was packed in a column, diameter of 2.5 cm, and provided for purification. After the column was buffered with 0.01M phosphate buffer (pH 7.2) containing 0.14M sodium chloride, 90 ml of the aforesaid solution containing the anti-tumor substance was passed therethrough at 4°C over 1 hour. After completion of the adsorption, the column was thoroughly washed with a fresh preparation of the same phosphate buffer and 50 mM of ethylamine-hydrochloride buffer (pH 11.0) was then passed through the column to elute the anti-tumor substance. The pH in the eluate was immediately adjusted to 7.4 with acetic acid. The amount of the recovered anti-tumor substance was 92% of that in the starting material. After concentration, the eluate, 2μg as the protein, was subjected to 15% SDS-polyacrylamide gel electrophoresis. The gel after the electrophoresis was stained with Coomassie Brilliant Blue to check the purity of the protein, whereby only a stained band was bound at the position corresponding to a molecular weight of 18,000 daltons as shown in Figure 4. The gel after the electrophoresis was minced into a rectangular shape vertical to the direction of electrophoresis. Thereafter each gel fragment was extracted with RPMI 1640 medium, and the cytotoxic activity of the gel fragments against L929 cells was determined by the method later described; the cytotoxic activity was detected only at the position identical to the Coomassie Brilliant Blue-stained band. These results confirmed that the anti-tumor substance, purity of at least 95% or higher, was contained in the fraction eluted from the monoclonal antibody-bound carrier.

## Example 3

The expression plasmid pM 307 obtained in Example 2 was digested with restriction enzymes Hpa I and Nru I to give a DNA fragment of about 1.1 kbp with the gene coding for the anti-tumor substance. The fragment was then ligated at the Hpa I restriction cleavage site of plasmid pPL-lambda (P-L Biochemicals) in the direction forwardly to the PL promoter to give an expression plasmid pM 315 (Figure 5).

Using the plasmid, lambda phage lysogen N 4830 strain was transformed in a conventional manner to give a transformant Escherichia coli N 4830/pM 315.

The transformant Escherichia coli N 4830/pM 315 was cultured at 30°C in mini jar fermenters - (Model MB-C, Kabushiki Kaisha Iwashiya Seibutsu Kagaku) charged with a 1 liter aliquot of M9 medium containing 100 μg/ml of ampicillin, 0.032 g/ml of tryptophane, 0.02 g/ml of yeast extract, 0.1 mM of $MgSO_4$ and 0.001 mM of $FeCl_3$. When the proliferation of the bacteria reached 1.0 in $OD_{550}$, an equivalent volume of the culture medium, prewarmed to 65°C, was added followed by an additional 4-hour culture at 42°C.

The resultant culture, 2 liters, was subjected to collection, lysis and purification in the same manner as in Example 2 to give the anti-tumor substance with a purity of at least 95% or higher.

## Example 4

Plasmid pM 301 was digested with restriction enzymes Pst I and Ava I to give a DNA fragment of about 400 bp having a non-translation region at the 5'-terminal end of genes coding both for the anti-tumor substance and for a signal peptide. The DNA fragment of 400 bp was further digested with restriction enzymes Hpa II to give a DNA fragment of about 220 bp that coded for the signal peptide.

Separately, plasmid pM 307 was cleaved with restriction enzymes Ava I and BamH I to give a DNA fragment of about 450 bp containing most of the genes that coded for the anti-tumor substance.

An oligonucleotide adaptor:

GATCCAAAGGACACCATGAGCACTGAAAGCATG-ATC

GTTTCCTGTGGTACTCGTGACTTTCGTACTAGGC

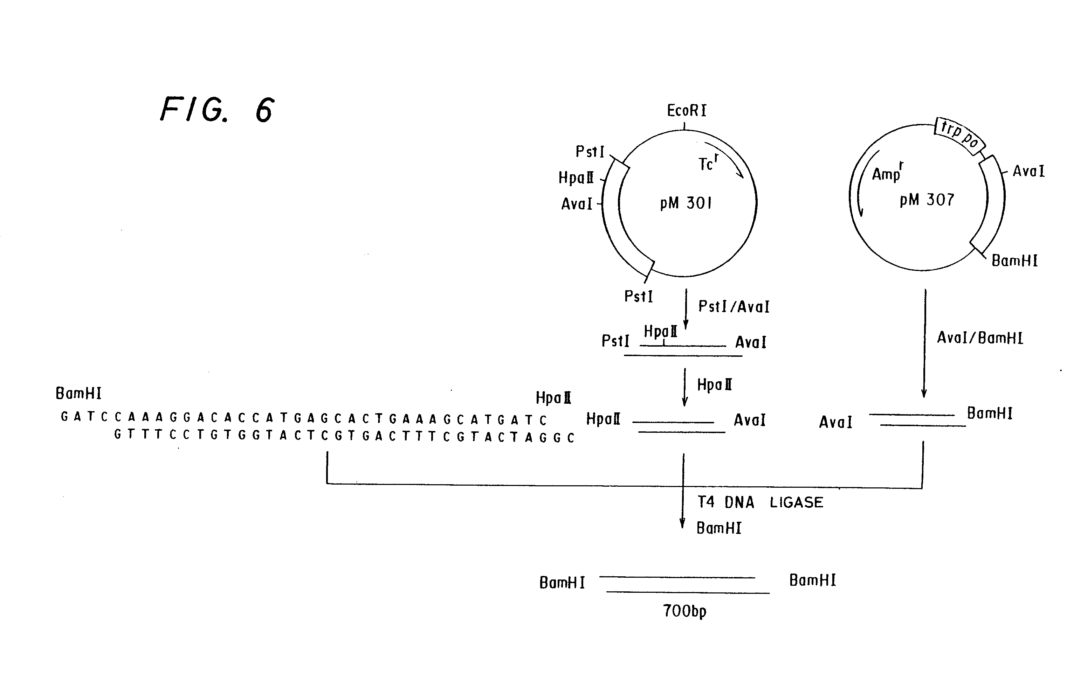

containing a protein synthesis initiation codon of the signal peptide chemically synthesized using the DNA synthesizer as described above was ligated with the aforesaid 220 bp DNA fragment and the 450 bp DNA fragment using T4 DNA ligase. The ligated product was redigested with restriction enzymes BamH I to give a DNA fragment of about 700 bp containing the whole translation regions of the anti-tumor substance (figure 6).

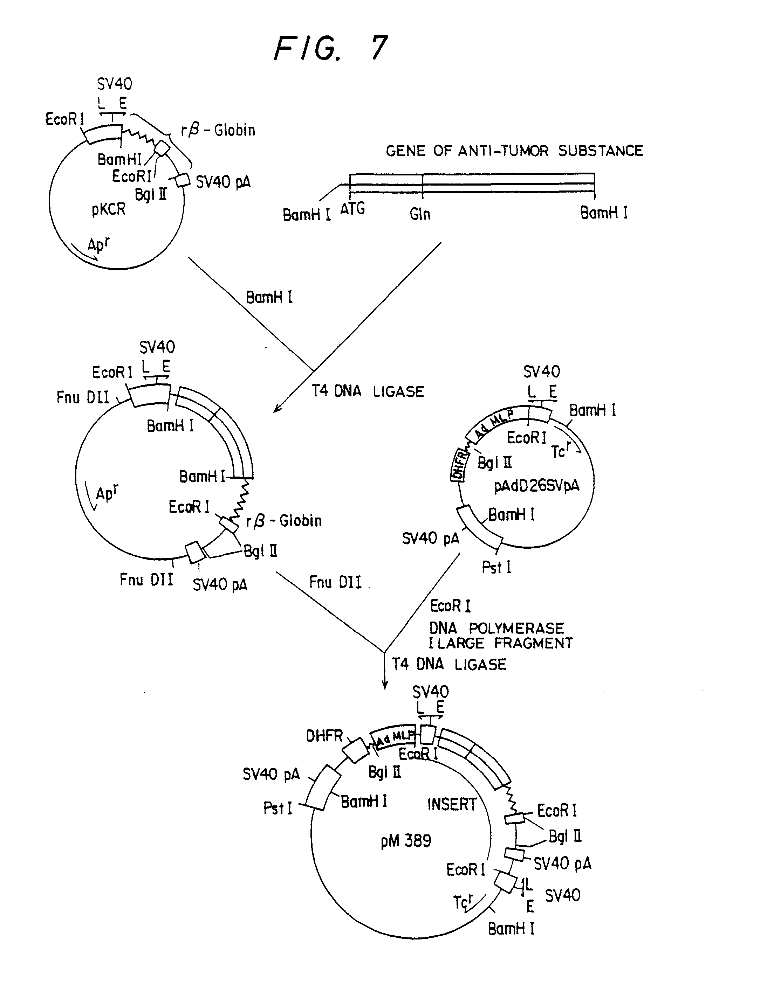

The DNA fragment was cloned to plasmid pKCR (11) and then to plasmid pAdD 26SVpA (12) in accordance with the method of Weissmann et al (13) to produce plasmid pM 389 that expressed the characteristic of the mammalian cell (Figure 7).

Dehydrofolic acid reductase-deficient Chinese hamster ovary (dhfr⁻CHO) cells (14) were transformed with plasmid pM 389 by the known calcium phosphate precipitation method (15).

Among the obtained dhfr⁺ cells, the clones showing a high cytotoxic activity were selected and proliferated in Dulbecco's MEM medium (Nissui Pharmaceutical Co., Ltd.) containing 0.5 $\mu$m of methotrexate (MTX) in accordance with the method of Weissmann et al (13). Clone CHO (DXB 11)/pM 389 resistant to 0.5 $\mu$M of MTX was cultured in Dulbecco's MEM medium to obtain $4 \times 10^6$ units of the anti-tumor substance in the supernatant obtained by centrifugation.

The supernatant was then purified in a manner similar that in Example 2 to obtain the anti-tumor substance with a purity of at least 95% or higher.

Example 5

Shuttle vector pAM 82 (16) of Escherichia coli and a yeast having acid phosphatase promoter were digested with restriction enzymes Xho I. An oligonucleotide adaptor:

TCGATGCAGGCAGTCAGATCATCTTCTCGAACC

ACGTCCGTCAGTCTAGTCGAAGAGCTTGGGGCT

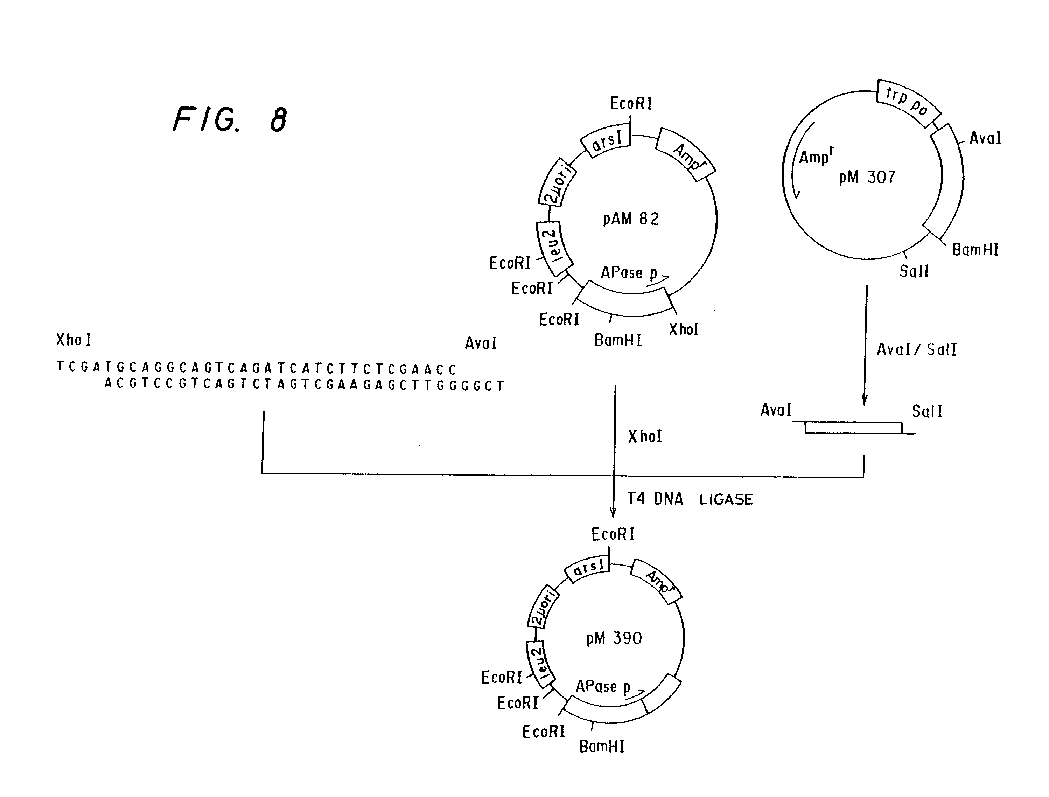

chemically synthesized by the DNA synthesizer, and the DNA fragment of about 750 bp coding for the anti-tumor substance digested with restriction enzymes Ava I and Sal I were ligated with the aforesaid vector using T4 DNA ligase to prepare expression plasmid pM 390 (Figure 8).

Using the plasmid, Saccharomyces cerevisiae AH 22 pho 80 strain (16) was transformed in accordance with the method of Hinnan et al (17) to give a Leu⁺ transformant AH 22 pho 80/pM 390. The transformant was proliferated at 30°C in phosphate-rich medium containing 20 $\mu$g/ml of histidine in accordance with the method of Matsubara et al (16). When the bacteria reached 0.5 in OD$_{610}$,

the culture medium was replaced with a phosphate-poor minimum medium followed by further incubation. When the bacteria reached 1.0 in OD$_{610}$, they were collected and treated at 30°C for 30 minutes in 1.2M sorbitol containing 100 $\mu$g/ml of thymolyase, 14 mM of $\beta$-mercaptoethanol and 50 mM of phosphate buffer (pH 7.2). The bacteria were collected by centrifugation and lysed in a mixture of 0.1% Triton X-100, 1mM of phenylmethylsulfonyl fluoride and 50 mM of phosphate buffer. The supernatant of the obtained lysate was passed through the monoclonal antibody-bound carrier similarly as in Example 2 to purify a fused protein containing the anti-tumor substance.

The fused protein is a protein binding an additional 7 amino acid:

Met-Arg-Ile-Arg-Thr-Thr-Ser

at the amino terminal end of the anti-tumor substance after cleaving the methyonyl bond with bromocyanide in a conventional manner (18). The fused protein was purified by gel filtration in a manner similar to that in Example 2 to give the anti-tumor substance with a purity of 95% or higher.

The obtained anti-tumor substance is typically characterized by the following properties:

a) molecular weight in the range of 16,000 to 20,000 daltons when measured with the SDS-polyacrylamide gel electrophoresis method, and a molecular weight of 30,000 to 45,000 daltons when measured with the gel filtration method;

b) cytotoxic activity against L929 cells and specific activity of $0.5 \times 10^9$ to $3 \times 10^9$ units/mg protein;

c) cytotoxic activity which is stable over a period of 18 hours at 4°C and pH 6.8 in an aqueous solution but declines to 10% of the initial activity upon 18 hours starting in an aqueous solution at pH 2.0 or lower.

The above properties were measured by the following methods.

Molecular weight

One $\mu$g of the purified anti-tumor substance was subjected to the SDS-polyacrylamide gel electrophoresis method as reported by Laemmli et al (19). A 15% polyacrylamide gel was stained with Coomassie Brilliant Blue after the completion of electrophoresis to determine its molecular weight, whereby a stained single band was noted and the molecular weight of the anti-tumor substance was determined by comparing the position of the band with that of the molecular weight marker (kit for measurement of molecular weight for electrophoresis, manufactured by Pharmacia Co., Ltd.). Although slight variation was noted during a plurality of measurements, the molecular weight was determined to be 16,000 to 20,000 daltons. The gel which had been simultaneously subjected to electrophoresis under the same conditions as for the staining but not stained with Coomassie Brilliant Blue was minced in a rectangular shape in the direction of the electrophoresis and each gel fraction was extracted with RPMI 1640 medium. The cytotoxic activity of each extract against L929 cells was determined. The substance exhibiting the cytotoxic activity was detected only at the position identical to that of the Coomassie Brilliant Blue-stained band.

The molecular weight of the purified anti-tumor substance was also measured by the gel filtration chromatography method. Sephadex G-75 - (Pharmacia Japan Co., Ltd.) was packed in a column, diameter of 1 cm, length of 1 m, and 100 $\mu$g of the anti-tumor substance was then fed into the column using as the developer 0.05M phosphate buffer (pH 7.5) containing 0.4M sodium chloride to effect gel filtration chromatography. After collecting 1 ml fractions of the eluate, the cytotoxic activity of each fraction against L929 cells was measured. The substance showing the cytotoxic activity was detected as a single peak and its molecular weight was determined to be 30,000 to 45,000 daltons, upon comparison with the position of the eluted molecular weight marker.

Specific activity

The anti-tumor substance exhibits a cytotoxic activity against L929 cells in vitro. The titer of the anti-tumor substance was determined with L929 cells as the target cells by the method reported by Eifel et al (20) with a slight modification. Particularly, mice-derived L929 cells (ATCC CCLI) purchased from ATCC were inoculated on a 96 well microplate (Nunc Co., Ltd.) together with 0.1 ml aliquots of a culture medium. After a lapse of 3 days, 0.1 ml of an actinomycin D solution, final concentration of 2 $\mu$g/ml, and 0.025 ml of serially diluted anti-tumor substance specimens were additionally placed in each well. On the day following the addition of the specimens, a neutral red solution, a pigment capable of being incorporated only by viable cells, was added in each well. After extracting the pigment incorporated by the cells the amount of the pigment was measured and then compared with that of the control well added with no specimen. The well showing 50% of the pigment amount of the control well was determined and the final concentration of the anti-tumor substance in the well was defined to be 50 units/ml. The amount of protein was measured with reference to human serum albumin standard using the protein measurement kit of Biorad Co., Ltd. according to Bradford's method (21) with a slight modification.

Based on the thus measured titer and protein amount, the specific activity of the anti-tumor substance was determined. Slight unevenness was noted among the measurements of the specific activity of the anti-tumor substance, but the value was $0.5 \times 10^9$ to $3 \times 10^9$ units/mg protein.

pH Stability

The stability of a purified anti-tumor substance in solutions was measured by monitoring the cytotoxic activity against L929 cells of the substance in buffer solutions of different pH values. The buffer solutions used were 5 kinds of 0.14M sodium chloride-containing 0.1M potassium chloride-hydrochloride buffer (pH 2.0), 0.14M sodium chloride-containing 0.1M citric acid-sodium citrate buffer (pH 4.0), 0.14M sodium chloride-containing 0.1M phosphate buffer (pH 7.0), 0.14M sodium chloride-containing 0.1M glycine-sodium hydroxide buffer (pH 9.0) and 0.14M sodium chloride-containing 0.1M glycine-sodium hydroxide buffer - (pH 11.0). After 4 ml of each buffer was mixed with 1 ml of a 0.14M sodium chloride solution containing 2 $\mu$g/ml of the purified anti-tumor substance, the mixture was allowed to stand at 4°C for 18 hours. A part of each mixture was taken out and its pH was measured. Then, the pH of the reminaing part of each mixture was adjusted to 7.0 using an aqueous solution of 0.5M sodium hydroxide or 0.5M hydrochloric acid. The cytotoxic activity of each mixture against L929 cells after neutralization was measured and compared with the remaining cytotoxic activity after correction of the solution amount upon neutralization followed by each treatment for pH. The anti-tumor substance was relatively stable at 4°C in an aqueous solution at pH 6.8 even after a lapse of 18 hours and thus, the value at pH 6.8 was designated 100%; the ratio of the remaining activity of the anti-tumor substance at each pH was shown below:

| pH | 2.0 | 10% or lower |
| pH | 4.0 | 21% |
| pH | 6.8 | 100% |
| pH | 8.9 | 85% |
| pH | 10.6 | 10% or lower. |

From the results, it was noted that the anti-tumor substance was stable in an aqueous solution, neutral or weakly alkaline, but had a tendency of losing its activity when it was allowed to stand either in an acidic aqueous solution of pH 4.0 or lower, or in a strongly alkaline solution over a long period of time.

Now the use of the anti-tumor substance will be more fully explained with reference to the following non-limiting Experimental Examples.

Experimental Example 1

One variety of the culture cells listed in Table 1 was suspended in 10% (V/V) bovine fetal serum-containing Eagle's MEM medium to give a concentration of $1 \times 10^5$/ml, and 0.1 ml of the suspension was inoculated on each well of a 96 well microplate. 0.1 ml aliquots of anti-tumor substance solution, serially diluted with the aforesaid medium, were added to each well, followed by culturing for 3 days at 37°C. After completion of the culturing, the concentration of the anti-tumor substance in wells showing the viable cell count of 50% as compared to the control well without addition of the anti-tumor substance was determined. The concentration of the anti-tumor substance required for 50% inhibition of the viable cell count in each cell line was as shown in Table 1.

### Table 1

| | |
|---|---|
| MKM-28 cell (human gastric cancer-derived cell line) | 500 units/ml |
| ACHN cell (human renal cancer-derived cell line) | 700 units/ml |
| MKN1 cell (human gastric cancer-derived cell line) | 1600 units/ml |
| MCF7 cell (human breast cancer-derived cell line) | 150 units/ml |
| SK-BR-3 cell (human breast cancer-derived cell line) | 350 units/ml |
| A549 cell (human lung cancer-derived cell line) | 2500 units/ml |
| SK-MEL-28 cell (human melanoma-derived cell line) | 450 units/ml |
| HEC-1C cell (human uterine cancer-derived cell line) | 1260 units/ml |
| MRC-5 cell (human fetal lung-derived normal cell line) | more than $2 \times 10^7$ units/ml |
| Flow 7000 (human fetal foreskin-derived normal cell line) | more than $2 \times 10^7$ units/ml |

As shown in Experimental Example 1, the anti-tumor substance exhibited a cytotoxic activity to the human cancer cell lines in the in vitro experimental system. However, the human normal cell lines were not affected by the anti-tumor substance. It was thus noted that the anti-tumor substance had a tendency to exhibit a strong cytotoxic activity against cancer cells as compared to that against normal cells.

The anti-tumor substance exhibits the anti-tumor activity not only in vitro, but also in vivo. This will be clarified by the following experimental examples.

Experimental Example 2 Anti-tumor effect on KB - (human nasopharyngeal cancer) cell-bearing nude mouse

$5 \times 10^6$ KB cells (ATCC CCL17) were subcutaneously transplanted to the abdomen of 7 week-old female nude mice (Shizuoka Experimental Animal Cooperative Association). After the formation of the tumor was confirmed 7 days later, a solution of the anti-tumor substance in 0.1% HSA - (human serum albumin)/saline was administered to the tail veins of one group of 5 mice with a dose of $2 \times 10^6$ units/body every third day, another group of 5 mice with $6 \times 10^5$ units/body daily to the tail vein and a third group of 5 mice with $2 \times 10^5$ units/body daily to the tumor. The tumor was isolated on the day following the final administration and its weight was measured and compared with that of the control group administered only with 0.1% HAS/saline. The results are shown in Tables 2-1 and 2-2.

Table 2-1

| Treatment | Dose | Weight of Tumor (g) |
|---|---|---|
| Control group | – | $1.7 \pm 0.1$ |
| Group intravenously administered with the anti-tumor substance | $2 \times 10^6$ units/body/every third day | $0.7 \pm 0.1$[*] |
| | $6 \times 10^5$ units/body/day | $1.2 \pm 0.1$[**] |

[*]    There is a stochastically significant difference within a significance level of 1% as compared to the control value.

[**]   There is a stochastically significant difference within a significance level of 5% as compared to the control value.

Table 2-2

| Treatment | Dose | Weight of Tumor (g) |
|---|---|---|
| Control group | – | $1.8 \pm 0.1$ |
| Group administered with the anti-tumor substance to the tumor | $2 \times 10^5$ units/body/day | $1.1 \pm 0.1$[*] |

[*]    There is a stochastically significant difference within a significance level of 1% as compared to the control value.

Experimental Example 3   Anti-tumor effect on MX-1 (human breast cancer) cell-bearing nude mouse

2 to 3 mm squares of MX-1 cells transferred subcutaneously with BALB/c nude mice were subcutaneously transplanted to the abdomen of 5 week-old female nude mice (Shizuoka Experimental Animal Cooperative Association). After the formation of the tumor was checked 12 days later, a solution of the anti-tumor substance in 0.1% HSA/saline was administered to the tail veins of one group of 5 mice in $2 \times 20^6$ units/body every third day, another group of 5 mice with $6 \times 10^5$ units/body daily to the tail vein and a third group of 5 mice with $2 \times 10^5$ units/body daily to the tumor. The tumor was isolated on the day following the final administration and its weight was measured and compared with that of the control group administered only with 0.1% HSA/saline. the results are shown in Tables 3-1 and 3-2.

Table 3-1

| Treatment | Dose | Weight of Tumor (g) |
|---|---|---|
| Control group | - | $2.2 \pm 0.2$ |
| Group intra-venously administered with the anti-tumor substance | $2 \times 10^6$ units/body/every third day | $0.5 \pm 0.2^*$ |
| | $6 \times 10^5$ units/body/day | $1.3 \pm 0.2^*$ |

\* There is a stochastically significant difference within a significance level of 1% as compared to the control value.

Table 3-2

| Treatment | Dose | Weight of Tumor (g) |
|---|---|---|
| Control group | - | $2.4 \pm 0.2$ |
| Group administered with the anti-tumor substance to the tumor | $2 \times 10^5$ units/body/day | $1.2 \pm 0.2^*$ |

\* There is a stochastically significant difference within a significance level of 1% as compared to the control value.

Experimental Example 4 Anti-tumor effect on Meth A (fibrosarcoma) cell-bearing (subcutaneous) mice

$2 \times 10^5$ Meth A cells were subcutaneously transplanted to the abdomen of 5 week-old male CBF1 mice (Shizuoka Experimental Animal Cooperative Association). After the formation of the tumor was checked after a lapse of 10 days, groups of 5 mice were administered a solution of the anti-tumor substance in 01.% HSA/saline intravenously in $6 \times 10^6$ units/body, to the tumor in $2 \times 10^6$ units/body and intramuscularly in $6 \times 10^6$ units/body, at once. Necrosis of the tumor was monitored 24 hours after administration. The tumor was isolated 3 days after the administration and its weight was measured and compared to that of the control group. The results are shown in Tables 4-1 to 4-3.

Table 4-1

| Treatment | Dose | Tumor Weight (g) | Rate of Necrosis Formation |
|-----------|------|------------------|---------------------------|
| Control group | - | $0.26 \pm 0.05$ | 0/5 |
| Group intra-venously administered with the anti-tumor substance | $6 \times 10^6$ units/body | $0.09 \pm 0.02^{**}$ | 4/5 |

**  There is a stochastically significant difference with in a significance level of 5% as compared to the control value.

Table 4-2

| Treatment | Dose | Tumor Weight (g) | Rate of Necrosis Formation |
|-----------|------|------------------|---------------------------|
| Control group | - | $0.28 \pm 0.04$ | 0/5 |
| Group administered with the anti-tumor substance to the tumor | $2 \times 10^6$ units/body | $0.12 \pm 0.01^{**}$ | 5/5 |

**  There is a stochastically significant difference within a significance level of 5% as compared to the control value.

Table 4-3

| Treatment | Dose | Tumor Weight (g) | Rate of Necrosis Formation |
|-----------|------|------------------|----------------------------|
| Control group | - | $0.25 \pm 0.05$ | 0/5 |
| Group intra-muscularly administered with the anti-tumor substance | $6 \times 10^6$ units/body | $0.10 \pm 0.05^{**}$ | 2/5 |

** There is a stochastically significant difference within a significance level of 5% as compared to the control value.

Experimental Example 5 Anti-tumor effect on Meth A cell bearing (intracutaneous) mice

2 x 10⁵ Meth A cells were intracutaneously transplanted to the abdomen of 5 week-old CBF1 mice (Shizuoka Experimental Animal Cooperative Association). After the formation of the tumor was checked after a lapse of 8 days, groups of 5 mice were administered intravenously with a solution of the anti-tumor substance in 0.1% HSA/saline in 2 x 10⁶ units/body, and to the tumor in 6 x 10⁵ units/body, at once, respectively. Necrosis of the tumor was monitored 24 hours after the administration. Further, the volume of the tumor was compared with that in the control group 20 days after the administration. The results are shown in Tables 5-1 and 5-2.

Table 5-1

| Treatment | Dose | Rate of Necrosis Formation | Volume of Tumor $(mm^3)$ | Cure Rate |
|---|---|---|---|---|
| Control group | - | 0/5 | $4280 \pm 243$ | 0/5 |
| Group intravenously administered with the anti-tumor substance | $2 \times 10^6$ units/body | 4/5 | $2171 \pm 884$ | 2/5 |

Table 5-2

| Treatment | Dose | Rate of Necrosis Formation | Volume of Tumor $(mm^3)$ | Cure Rate |
|---|---|---|---|---|
| Control group | - | 0/5 | $5280 \pm 725$ | 0/5 |
| Group administered with the anti-tumor substance to the tumor | $2 \times 10^6$ units/body | 5/5 | $350 \pm 281$[*] | 4/5 |
|  | $6 \times 10^5$ units/body | 5/5 | $1973 \pm 879$[**] | 3/5 |

[*] There is a stochastically significant difference within a significance level of 1% as compared to the control value.

[**] There is a stochastically significant difference within a significance level of 5% as compared to the control value.

Experimental Example 6  Anti-tumor effect on Lewis lung cancer-bearing mouse

$5 \times 10^5$ Lewis lung cancer cells were subcutaneously transplanted to the abdomen of 6 week-old male C57BL/6 mice. After the formation of tumors was checked after a lapse of 9 days, a solution of the anti-tumor substance in 0.1% HSA/saline was administered to groups of 5 mice intravenously or to the tumor, respectively, in $2 \times 10^6$ units/body at once. Necrosis of the tumor was macroscopically monitored 24 hours after administration. The results are shown in Tables 6-1 and 6-2.

Table 6-1

| Treatment | Dose | Rate of Necrosis Formation |
|---|---|---|
| Control group | - | 0/5 |
| Group intra-venously administered with the anti-tumor substance | $2 \times 10^6$ units/body | 4/5 |

Table 6-2

| Treatment | Dose | Rate of Necrosis Formation |
|---|---|---|
| Control group | - | 0/5 |
| Group admini-stered with the anti-tumor substance | $2 \times 10^6$ units/body | 5/5 |

Experimental Example 7  Acute Toxicity

The purified anti-tumor substance was dissolved in saline. The solution of the anti-tumor substance was intravenously administered to a group of 10 each of laboratory animals of 6 week-old-female and -male ICR type mice, and a group of 10 each of 6 week-old-female and -male Wistar rats (both by Japan Charles River Co., Ltd.) in various concentrations. After monitoring for 2 weeks, $LD_{50}$ was determined. As a result, the $LD_{50}$ value was higher than $1 \times 10^{10}$ units/kg in both mice and rats.

As is evident from the foregoing experimental examples, the anti-tumor substance of the present invention is safe and extremely useful since it shows anti-tumor effects on various cancers and the acute toxicity test reveals that the $LD_{50}$ value of the substance is much greater than the dose that exhibits the anti-tumor effects.

The anti-tumor substance shows an anti-tumor effect on the aforesaid tumor cells. Therefore, the anti-tumor substance can be used in various desired applications, depending upon the kind of tumor or the tumor site in the organ that bears tumor tissues to be treated. Application methods include oral and parenteral administration; the parenteral administration includes intravenous administration, intraarterial administration, intramuscular administration, subcutaneous administration, intraepidermal administration, intralymph node administration, intracorporeal administration, and the like. Among these applications, specifically suited are intravenous administration, intraarterial administration, intramuscular administration, subcutaneous administration and intracorporeal administration.

The medical preparations which are usable in the invention include injection, eyedrop, inhalation, and medicines for external application. The anti-tumor substance can be used as medicine for oral administration, medicine for rectal administration and medicine for intravaginal administration.

It is expected that the optimum dose of the anti-tumor substance would greatly vary dependent upon the condition of the patient, the kind of tumor, the manner of application, etc., but a therapeutic dose for an adult is $10^3$ to $10^9$ units/day, preferably $10^4$ to $10^9$ units/day in the systemic administration by intravenous administration, intraarterial administration, intramuscular administration, etc., $10^5$ to

$10^9$ units/day in the oral administration, and $10^3$ to $10^8$ units/day in the local administration. The dose can appropriately be varied depending upon the application path and/or the patient's condition.

The anti-tumor substance can be prepared into a medical preparation in a conventional manner together with an optional pharmaceutical vehicle, base and/or excipient. The anti-tumor substance can be used in oil-soluble capsules or tablets for oral administration, in water-soluble injections or injections of lyophilized powders for injections, in rectal suppositories for rectal administration, and in ointments or lotions for medicine for external use. In the preparation of the medical composition of the invention, conventional vehicles can be used, desirably including, human serum albumin, sucrose, gelatin and/or polyethylene glycol.

Concrete applications of anti-tumor substances of the present invention as described above are explained below referring to examples in pharmaceutical use. the present invention is, however, not to be limited to any of the applications and/or examples set forth in this specification.

## Application Example 1 Water-soluble injection

$10^9$ units of the anti-tumor substance were dissolved in 100 ml of 0.14M sodium chloride-containing 0.01M phosphate buffer (pH 7.0) prepared using distilled water for injection. The solution was filtered under sterile conditions with a membrane filter and 1 ml aliquots of the resultant filtrate were filled into glass containers which were then sealed to obtain the product.

## Application Example 2 Lyophilized injection

$10^9$ units of the anti-tumor substance were dissolved in 100 ml of 0.14M sodium chloride-containing 0.01M phosphate buffer (pH 7.0) prepared using distilled water for injection. Thereafter, 1 g of human serum albumin was added to the solution to give a total amount of 200 ml. The solution was filtered under sterile conditions using a membrane filter and 2 ml aliquots of the filtrate were charged into glass containers, followed by lyophilization. After completion of the lyophilization, the containers were sealed to obtain the product.

## Application Example 3 Ointment

10 mg of the anti-tumor substance were weighed and kneaded with 1 ml of liquid paraffin. Thereafter 50 g of Vaseline(R) were added thereto and the mixture was mixed. Further 50 g of Vaseline(R) were added and the mixture was mixed to homogeneity to obtain an ointment.

While the invention has been described in detail and with reference to specific embodiments thereof, it will be apparent to one skilled in the art that various changes and modifications can be made therein without departing from the spirit and scope thereof.

## List of Publications

1. Miyoshi, I., Hiraki, S., Tsubota, T., Kubonishi, I., Matsuda, Y., Nakayama, T., Kishimoto, H. and Kimura, I., Nature 267, 843 (1977);

2. Maniatis, T., Fritsch, E.F. and Sambrook, J., in Molecular Cloning, A Laboratory Manual, page 213 (Cold Spring Harbor Laboratory, New York 1982);

3. Land, H., Grez, M., Hauser, H., Lindenmaier, W. and Schutz, G., Nucleic Acids Res., 9, 2251 (1981);

4. Okayama, H. and Berg, P., Mol. Cell. Biol., 2, 161 (1982);

5. Hewick, R.M., Hunkapiller, M.W., Hood, L.E. and Dreyer, W.J., J. Biol. Chem., 256, 7990 - (1981);

6. Maniatis, T., Fritsch, E.F. and Sambrook, J., in Molecular Cloning, A Laboratory Manual, page 309 (Cold Spring Harbor Laboratory, New York 1982);

7. Messing, J., Crea, R. and Seeburg, P.H., Nucleic Acids Res., 9, 309 (1981);

8. Maxam, A.M. and Gilbert, W., Proc. Natl. Acad. Sci. USA, 74, 560 (1977);

9. Köhler, G. and Milstein, C., Nature, 256, 495 (1975);

10. Dunau, M.L. and Goldman, D., Anal. Biochem., 110, 201 (1981);

11. O'Hara, K., Benoist, C. and Breathnach, R., Proc, Natl. Acad. Sci. USA, 78, 1527 (1981);

12. Kaufman, R.J. and Sharp, P.A., J. Mol. Biol., 159, 601 (1982);

13. Haynes, J. and Weissmann, C., Nucleic Acids Res., 11, 687 (1983);

14. Urlaub, G. and Chasin, L.A., Proc. Natl. Acad. Sci. USA, 77, 4216 (1980);

15. Graham, F.L. and van der Ed, A., J. Virology, 52, 456 (1973);

16. Miyanohara, A., Toh-e, A., Nozaki, C., Hamada, F., Ohtomo, N. and Matsubara, K., Proc. Natl. Acad. Sci. USA, 80, 1 (1983);

17. Hinnen, A., Hicks, J.B. and Fink, G.R., Proc. Natl. Acad. Sci. USA, 75, 1929 (1978);

18. Koide, T., SEIKAGAKU JIKKEN KOZA - (Biochemical Experiment Series), 1, Chemistry of Protein II, Published by Tokyo Kagaku Dojin Publishing Co., Ltd., Page 235 (1976);

19. Laemmli, U.K., Nature, 227, 680 (1970);

20. Eifel, P.J., Walker, S.M. and Lucas, Z.J., Cell. Immunol., 15, 208 (1975);

21. Bradford, M.M., Anal. Biochemi., 72, 248 (1976).


Microorganisms

Among the microorganisms used in the present invention, Escherichia coli HB 101/pM 307 and Escherichia coli N 4830/pM 315 have been deposited in the Fermentation Research Institute respectively under Deposit Nos. 8337 and 8336. Further, Notice of Refusal to take charge of deposit has been received with plasmids pTM 1, pM 307, pM 315, pM 389, CHO(DXB11)/pM 389 and pM 390, as being microorganisms outside the scope put under the custody, but these plasmids are being maintained available to the public.


**Claims**

1. An amino acid composition, comprising an amino acid sequence of:

H-Gln Ala Val Arg Ser Ser Ser Arg Thr Pro Ser Asp Lys Pro Val Ala His Val Val Ala Asn Pro Gln Ala Glu Gly Gln Leu Gln Trp Leu Asn Arg Arg Ala Asn Ala Leu Leu Ala Asn Gly Val Glu Leu Arg Asp Asn Gln Leu Val Val Pro Ser Glu Gly Leu Tyr Leu Ile Tyr Ser Gln Val Leu Phe Lys Gly Gln Gly Cys Pro Ser Thr His Val Leu Leu Thr His Thr Ile Ser Arg Ile Ala Val Ser Tyr Gln Thr Lys Val Asn Leu Leu Ser Ala Ile Lys Ser Pro Cys Gln Arg Glu Thr Pro Glu Cly Ala Glu Ala Lys Pro Trp Tyr Glu Pro Ile Tyr Leu Gly Gly Val Phe Gln Leu Glu Lys Gly Asp Arg Leu Ser Ala Glu Ile Asn Arg Pro Asp Tyr Leu Asp Phe Ala Glu Ser Gly Gln Val Tyr Phe Gly Ile Ile Ala Leu-OH.

2. The composition of claim 1, which shows a molecular weight in the range of 16,000 to 20,000 daltons upon SDS-polyacrylamide gel electrophoresis, and a molecular weight in the range of 30,000 to 45,000 daltons upon gel filtration.

3. The composition of claim 1, which has a specific activity in the range of 0.5 x 10⁹ to 3 x 10⁹ units/mg protein.

4. The composition of claim 1, wherein said composition additionally contains the amino acid sequence of Met-Arg-Ile-Arg-Thr-Thr-Ser.

5. A process for producing an amino acid composition having the amino acid sequence of claim 1, said process comprising the steps:

extracting messenger RNA (mRNA) coding for said amino acid composition from a cell that is capable of producing said amino acid composition;

preparing a double-stranded complementary DNA - (cDNA) using said mRNA as a template;

inserting said cDNA into an expression vector;

transforming a host cell with said expression vector; and

culturing the transformed host cell.

6. the process of claim 5, wherein said amino acid composition shows a molecular weight in the range of 16,000 to 20,000 daltons upon SDS-polyacrylamide gel electrophoresis, and a molecular weight in the range of 30,000 to 45,000 daltons upon gel filtraiton.

The process of claim 5, wherein said amino acid composition has a specific activity in the range of 0.5 x 10⁹ to 3 x 10⁹ units/mg protein.

8 The process of claim 5, wherein said amino acid composition additionally contains the amino acid sequence of Met-Arg-Ile-Arg-Thr-Thr-Ser.

9. The process of claim 5, wherein said cell capable of producing said amino acid composition is a human lymphatic cell.

10. The process of claim 9, wherein said human lymphatic cell is a BALL-1 cell.

11. The process of claim 9, wherein said human lymphatic cell has been exposed to the action of a stimulatory agent.

12. The process of claim 5, wherein said host cell is of the species Escherichia coli.

13. The process of claim 5, wherein said expression vector is a plasmid.

14. The process of claim 5, wherein said cDNA contains the base sequence of

CAG GCA GTC AGA TCA TCT TCT CGA ACC
CCG AGT GAC AAG CCT GTA GCC CAT GTT
GTA GCA AAC CCT CAA GCT GAG GGG CAG
CTC CAG TGG CTG AAC CGC CGG GCC AAT
GCC CTC CTG GCC AAT GGC GTG GAG CTG
AGA GAT AAC CAG CTG GTG GTG CCA TCA
GAG GGC CTG TAC CTC ATC TAC TCC CAG
GTC CTC TTC AAG GGC CAA GGC TGC CCC
TCC ACC CAT GTG CTC CTC ACC CAC ACC
ATC AGC CGC ATC GCC GTC TCC TAC CAG
ACC AAG GTC AAC CTC CTC TCT GCC ATC

AAG AGC CCC TGC CAG AGG GAG ACC CCA
GAG GGG GCT GAG GCC AAG CCC TGG TAT
GAG CCC ATC TAT CTG GGA GGG GTC TTC
CAG CTG GAG AAG GGT GAC CGA CTC AGC
GCT GAG ATC AAT CGG CCC GAC TAT CTC
GAC TTT GCC GAG TCT GGG CAG GTC TAC
TTT GGG ATC ATT GCC CTG TGA.

15. The process of claim 5, wherein said culturing step additionally contains the steps of:

recovering said amino acid composition from a resultant culture; and

purifying said amino acid composition with a monoclonal antibody which is specific to said amino acid composition.

16. A medical composition, comprising a pharmaceutically acceptable carrier and a pharmaceutically effective amount of an amino acid composition having the amino acid sequence of claim 1.

17. The medical composition of claim 16, wherein said amino acid composition shows a molecular weight in the range of 16,000 to 20,000 daltons upon SDS-polyacrylamide gel electrophoresis, and a molecular weight in the range of 30,000 to 45,000 daltons upon gel filtration.

18. The medical composition of claim 17, wherein said pharmaceutically effective amount is a tumor treating efffective amount.

19. The medical composition of claim 17, wherein said composition is in an orally or parenterally administrable form.

20. The use of the amino acid composition according to claims 1 to 4 in the treatment of tumor.

21. The use according to claim 20, wherein said tumor is gastric cancer, renal cancer, breast cancer, lung cancer, melanoma, uterine cancer, nasopharyngeal cancer of fibrosarcoma tumors.

22. The use according to claim 20, wherein said composition is administered parenterally.

23. The use according to claim 22, wherein said amount is $10^3$ to $10^9$ units per day.

24. The use according to claim 20, wherein said composition is administered orally.

25. The use according to claim 24, wherein said amount is $10^5$ to $10^9$ units per day.

26. The use according to claim 20, wherein said composition is administered locally.

27. The use according to claim 26, wherein said amount is $10^3$ to $10^8$ units per day.

Claims for Austria 1. A process for producing an amino acid composition, comprising an amino acid sequence of:

H-Gln Ala Val Arg Ser Ser Ser Arg Thr Pro Ser Asp

Lys Pro Val Ala His Val Val Ala Asn Pro Gln Ala
Glu Gly Gln Leu Gln Trp Leu Asn Arg Arg Ala Asn
Ala Leu Leu Ala Asn Gly Val Glu Leu Arg Asp Asn
Gln Leu Val Val Pro Ser Glu Gly Leu Tyr Leu Ile
Tyr Ser Gln Val Leu Phe Lys Gly Gln Gly Cys Pro
Ser Thr His Val Leu Leu Thr His Thr Ile Ser Arg Ile
Ala Val Ser Tyr Gln Thr Lys Val Asn Leu Leu Ser
Ala Ile Lys Ser Pro Cys Gln Arg Glu Thr Pro Glu
Cly Ala Glu Ala Lys Pro Trp Tyr Glu Pro Ile Tyr
Leu Gly Gly Val Phe Gln Leu Glu Lys Gly Asp Arg
Leu Ser Ala Glu Ile Asn Arg Pro Asp Tyr Leu Asp
Phe Ala Glu Ser Gly Gln Val Tyr Phe Gly Ile Ile Ala
Leu-OH.

said process comprising the steps:

extracting messenger RNA (mRNA) coding for said amino acid composition from a cell that is capable of producing said amino acid composition;

preparing a double-stranded complementary DNA - (cDNA) using said mRNA as a template;

inserting said cDNA into an expression vector;

transforming a host cell with said expression vector;

and culturing the transformed host cell.

2. The process of claim 1, wherein said amino acid composition shows a molecular weight in the range of 16,000 to 20,000 daltons upon SDS-polyacrylamide gel electrophoresis, and a molecular weight in the range of 30,000 to 45,000 daltons upon gel filtration.

3. The process of claim 1, wherein said amino acid composition has a specific activity in the range of $0.5 \times 10^9$ to $3 \times 10^9$ units/mg protein.

4. The process of claim 1, wherein said amino acid composition additionally contains the amino acid sequence of Met-Arg-Ile-Arg-Thr-Thr-Ser.

5. The process of claim 1, wherein said cell capable of producing said amino acid composition is a human lymphatic cell.

6. The process of claim 5, wherein said human lymphatic cell is a BALL-1 cell.

7. The process of claim 5, wherein said human lymphatic cell has been exposed to the action of a stimulatory agent.

8. The process of claim 1, wherein said host cell is of the species Escherichia coli.

9. The process of claim 1, wherein said expression vector is a plasmid.

10. The process of claim 1, wherein said cDNA contains the base sequence of:

CAG GCA GTC AGA TCA TCT TCT CGA ACC
CCG AGT GAC AAG CCT GTA GCC CAT GTT

GTA GCA AAC CCT CAA GCT GAG GGG CAG
CTC CAG TGG CTG AAC CGC CGG GCC AAT
GCC CTC CTG GCC ATT GGC GTG GAG CTG
AGA GAT AAC CAG CTG GTG GTG CCA TCA
GAG GGC CTG TAC CTC ATC TAC TCC CAG
GTC CTC TTC AAG GGC CAA GGC TGC CCC
TCC ACC CAT GTG CTC CTC ACC CAC ACC
ATC AGC CGC ATC GCC GTC TCC TAC CAG
ACC AAG GTC AAC CTC CTC TCT GCC ATC
AAG AGC CCC TGC CAG AGG GAG ACC CCA
GAG GGG GCT GAG GCC AAG CCC TGG TAT
GAG CCC ATC TAT CTG GGA GGG GTC TTC
CAG CTG GAG AAG GGT GAC CGA CTC AGC
GCT GAG ATC AAT CGG CCC GAC TAT CTC
GAC TTT GCC GAG TCT GGG CAG GTC TAC
TTT GGG ATC ATT GCC CTG TGA

11. The process of claim 1, wherein said culturing step additionally contains the steps of:

recovering said amino acid composition from a resultant culture; and

purifying said amino acid composition with a monoclonal antibody which is specific to said amino acid composition.

12. A process for preparing a medical composition, characterized in that a pharmaceutically acceptable carrier and a pharmaceutically effective amount of an amino acid composition prepared according to claim 1 are mixed.

13. A process according to claim 12, wherein said amino acid composition shows a molecular weight in the range of 16,000 to 20,000 daltons upon SDS-polyacrylamide gel electrophoresis, and a molecuar weight in the range of 30,000 to 45,000 daltons upon gel filtration.

14. A process according to claim 12, wherein said pharmaceutically effective amount is a tumor treating effective amount.

15. The use according to claim 12, wherein said composition is in a orally or parenterally administerable form.

16. The use of the amino acid composition prepared according to claims 1 to 11 in the treatment of tumor.

17. The use according to claim 16, wherein said tumor is gastric cancer, renal cancer, breast cancer, lung cancer, melanoma, uterine cancer, nasopharyngeal cancer or fibrosarcoma tumors.

18. The use according to claim 16, wherein said composition is administered parenterally.

19. The use according to claim 18, wherein said amount is $10^3$ to $10^9$ units per day.

20. The use according to claim 18, wherein said composition is administered orally.

21. The use according to claim 20, wherein said amount is $10^5$ to $10^9$ units per day.

22. The use according to claim 16, wherein said composition is administered locally.

23. The use of claim 22, wherein said amount is $10^3$ to $10^8$ units per day.

0 211 321

# FIG. 1

FIG. 2

GCAGAGGACCAGCTAACAGGGAGAGAAGCAACTACAGACCCCCCCTGAAAA
CAACCCTCAGACGCCACATCCCCTGACAAGCTGCCAGGCAGGTTCTCTTCC
TCTCACATACTGACCCACGGCTCCACCCTCTCTCCCCTGGAAAGGACACC

ATG AGC ACT GAA AGC ATG ATC CGG GAC GTG GAG CTG GCC

GAG GAG GCG CTC CCC AAG AAG ACA GGG GGG CCC CAG GGC

TCC AGG CGG TGC TTG TTC CTC AGC CTC TTC TCC TTC CTG

ATC GTG GCA GGC GCC ACC ACG CTC TTC TGC CTG CTG CAC

TTT GGA GTG ATC GGC CCC CAG AGG GAA GAG TTC CCC AGG

GAC CTC TCT CTA ATC AGC CCT CTG GCC CAG GCA GTC AGA

TCA TCT TCT CGA ACC CCG AGT GAC AAG CCT GTA GCC CAT

GTT GTA GCA AAC CCT CAA GCT GAG GGG CAG CTC CAG TGG

CTG AAC CGC CGG GCC AAT GCC CTC CTG GCC AAT GGC GTG

GAG CTG AGA GAT AAC CAG CTG GTG GTG CCA TCA GAG GGC

CTG TAC CTC ATC TAC TCC CAG GTC CTC TTC AAG GGC CAA

GGC TGC CCC TCC ACC CAT GTG CTC CTC ACC CAC ACC ATC

AGC CGC ATC GCC GTC TCC TAC CAG ACC AAG GTC AAC CTC

CTC TCT GCC ATC AAG AGC CCC TGC CAG AGG GAG ACC CCA

GAG GGG GCT GAG GCC AAG CCC TGG TAT GAG CCC ATC TAT

CTG GGA GGG GTC TTC CAG CTG GAG AAG GGT GAC CGA CTC

AGC GCT GAG ATC AAT CGG CCC GAC TAT CTC GAC TTT GCC

GAG TCT GGG CAG GTC TAC TTT GGG ATC ATT GCC CTG TGA

GGAGGACGAACATCCAACCTTCCCAAACGCCTCCCCTGTCCCAATCCCTTT
ATTACCCCCTCCTTCAGACACCCTCAACC TCTTCTGGCTCAAAAAGAGAA
TTGGGGGCTTAGGGTCGGAACCCAAGCTTAGAACTTTAAGCAACAAGACCA
CCACTTCGAAACCTGGGATTCAGGAATGTGTGGCCTGCACAGTGAAGTGCT
GGCAACCACTAAGAATTCAAACTGGGGCCTCCAGAACTCACTGGGGCCTAC
AGCTTTGATCCCTGACATCTGGAATCTGGAGACCAGGGAGCCTTTGGTTCT
GACCAGAATGCTGCAGGACTTGAGAAGACCTCACCTAGAAATTGACACAAG
TGGACCTTAGGCCTTCCTCTCTCCAGATGTTTCCAGACTTCCTTGAGACAC
GGAGCCCAGCCCTCCCCATGGAGCCAGCTCCCTCTATTTATGTTTGCACTT
GTGATTATTTATTATTTATTTATTATTTATTTATTTACAGATGAATGTATT
TATTTGGGAGACCGGGGTATCCTGGGGGACCCAATGTAGGAGCTGCCTTGG
CTCAGACATGTTTTCCGTGAAAACGGAGCTGAACAATAGGCTGTTCCCATG
TAGCCCCCTGGCCTCTGTGCCTTCTTTTGATTATGTTTTTTAAAATATTTA
TCTGATTAAGTTGTCTAAACAATGCTGATTTGGTGACCAACTGTCACTCAT
TGCTGAGCCTCTGCTCCCCAGGGGAGTTGTGTCTGTAATCGCCCTACTATT
CAGTGGCGAGAAATAAAGTTTGCTT

# FIG. 3

# FIG. 4

MOLECULAR WEIGHT MARKER          ANTI-TUMOR SUBSTANCE

PHOSPHORYLASE b
(MW 94,000)

BOVINE SERUM ALBUMIN
(MW 67,000)

OVALBUMIN
(MW 45,000)

CARBONIC ANHYDRASE
(MW 30,000)

SOYBEAN TRYPSIN INHIBITOR
(MW 20,100)

α-LACTALBUMIN
(MW 14,400)

# F I G. 5

FIG. 6

EcoRI

PstI
HpaII
AvaI

Tc^r

pM 301

PstI

PstI/AvaI

PstI    HpaII    AvaI

HpaII

BamHI

HpaII
GATCCAAAGGACACCATGAGCACTGAAAGCATGATC
GTTTCCTGTGGTACTCGTGACTTTCGTACTAGGC

HpaII ——— AvaI

trp po

Amp^r    pM 307    AvaI

BamHI

AvaI/BamHI

AvaI ——— BamHI

T4 DNA LIGASE
BamHI

BamHI ——————— BamHI
700bp

0 211 321

# FIG. 7

FIG. 8

XhoI                                                          AvaI

TCGATGCAGGCAGTCAGATCATCTTCTCGAACC
    ACGTCCGTCAGTCTAGTCGAAGAGCTTGGGGCT

0 211 321